(19) Europäisches Patentamt
European Patent Office
Office européen des brevets

(11) Veröffentlichungsnummer: **0 192 055 B1**

(12) # EUROPÄISCHE PATENTSCHRIFT

(45) Veröffentlichungstag der Patentschrift: **09.08.89**

(51) Int. Cl.[4]: **C 07 D233/60**, C 07 D249/08, A 01 N 43/50, A 01 N 43/653

(21) Anmeldenummer: **86100632.8**

(22) Anmeldetag: **30.05.83**

(60) Veröffentlichungsnummer der früheren Anmeldung nach Art. 76 EPÜ: **0096786**

(54) **Hydroxyalkinyl-azolyl-Derivate, Verfahren zu ihrer Herstellung sowie ihre Verwendung als Fungizide.**

(30) Priorität: **12.06.82 DE 3222191**

(43) Veröffentlichungstag der Anmeldung: **27.08.86 Patentblatt 86/35**

(45) Bekanntmachung des Hinweises auf die Patenterteilung: **09.08.89 Patentblatt 89/32**

(84) Benannte Vertragsstaaten: **AT BE CH DE FR GB IT LI NL SE**

(56) Entgegenhaltungen:
**EP-A- 0 033 501**
**EP-A- 0 052 424**
**EP-A- 0 071 009**
**EP-A- 0 097 425**
**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Jäger, Gerhard, Dr.**
**Paul-Klee-Strasse 28j**
**D-5090 Leverkusen 1 (DE)**
Erfinder: **Böckmann, Klaus, Dr.**
**Andreas-Gryphius-Strasse 7**
**D-5000 Köln 80 (DE)**
Erfinder: **Büchel, Karl Heinz, Prof. Dr.**
**Dabringhausener Strasse 42**
**D-5093 Burscheid (DE)**
Erfinder: **Brandes, Wilhelm, Dr.**
**Eichendorffstrasse 3**
**D-5653 Leichlingen (DE)**
Erfinder: **Frohberger, Paul-Ernst, Dr.**
**Willi-Baumeister-Strasse 5**
**D-5090 Leverkusen 1 (DE)**
Erfinder: **Scheinpflug, Hans, Dr.**
**Am Thelenhof 15**
**D-5090 Leverkusen 1 (DE)**

**EP 0 192 055 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Hydroxyalkinylazolyl-Derivate, mehrere Verfahren zu ihrer Herstellung sowie ihre Verwendung als Fungizide.

Es ist bereits bekannt geworden, daß bestimmte Hydroxyalkyl-triazole, wie beispielsweise 2-(4-Biphenylyl)-1-(2,4-dichlorphenyl)-3-(1,2,4-triazol-1-yl)-2-propanol oder 2-(Biphenylyl)-1-(4-chlorphenyl)-3-(1,2,4-triazol-1-yl)-2-propanol, fungizide Eigenschaften aufweisen (vergleiche DE-A-2 920 374). Die Wirksamkeit dieser Verbindungen ist jedoch, insbesondere bei niedrigen Aufwandmengen und -konzentrationen, nicht immer ganz befriedigend.

Außerdem sind aus der US-A-4 413 003 und der US-A-4 414 210 fungizid wirksame Hydroxyalkinyl-azolyl-Derivate bekannt, in denen das Carbinol-Kohlenstoffatom einen durch Fluor substituierten Phenyl-Rest trägt. Es werden jedoch keine Verbindungen offenbart, in denen an dem betreffenden Phenyl-Rest andere Substituenten als Fluor vorhanden sind.

Es wurden neue Hydroxyalkinyl-azolyl-Derivate der allgemeinen Formel I

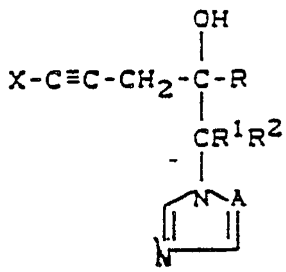

(I)

in welcher

A für ein Stickstoffatom oder eine CH-Gruppe steht,

R für Phenyl steht, das gegebenenfalls einfach bis zweifach, gleich oder verschieden substituiert ist durch Chlor, Methyl, Trifluormethyl sowie gegebenenfalls durch Chlor substituiertes Phenyl,

$R^1$ für Wasserstoff, Methyl, Vinyl, Allyl, Propargyl sowie für Benzyl steht, das im Phenylteil gegebenenfalls einfach bis zweifach, gleich oder verschieden substituiert ist durch Fluor, Chlor, Brom, Methyl, Ethyl, Isopropyl, tert.-Butyl, Methoxy, Methylthio, Ethoxy, Trifluormethyl, Trifluormethoxy, Trifluormethylthio, Nitro, Dimethylamino, Methoxycarbonyl sowie gegebenenfalls durch Chlor substituiertes Phenyl,

$R^2$ für Wasserstoff oder Methyl steht und

X für Wasserstoff, Brom oder Jod steht,

sowie deren Säureadditions-Salze und Metallsalz-Komplexe gefunden.

Weiterhin wurde gefunden, daß man die Hydroxyalkinylazolyl-Derivate der allgemeinen Formel I sowie deren Säureadditions-Salze und Metallsalz-Komplexe erhält, wenn man

a) Azolyl-ketone der allgemeinen Formel II

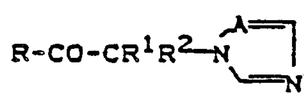

(II)

in welcher A, R, $R^1$ und $R^2$ die oben angegebene Bedeutung haben, mit Propargylhalogeniden der allgemeinen Formel III

$$HC \equiv C—CH_2—Hal \qquad (III)$$

in welcher Hal für Chlor oder Brom steht, in Gegenwart von aktiviertem Aluminium und in Gegenwart eines Verdünnungsmittels umsetzt, oder

b) Hydroxyalkinyl-halogenide der allgemeinen Formel IV

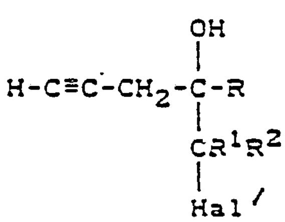

(IV)

in welcher R, $R^1$ und $R^2$ die oben angegebene Bedeutung haben und Hal für Chlor oder Brom steht, mit Azolen der allgemeinen Formel V

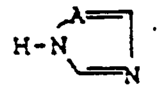

(V)

in welcher A die oben angegebene Bedeutung hat, in Gegenwart einer Base und in Gegenwart eines Verdünnungsmittels umsetzt, oder

c) Oxirane der allgemeinen Formel VI

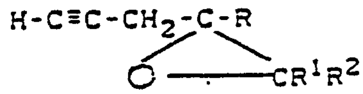

(VI)

in welcher R, $R^1$ und $R^2$ die oben angegebene Bedeutung haben, mit Azolen der allgemeinen Formel V

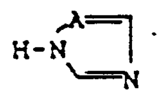

(V)

in welcher A die oben angegebene Bedeutung hat, in Gegenwart eines Verdünnungsmittels und gegebenenfalls in Gegenwart einer Base umsetzt, oder

d) gegebenenfalls die nach den Verfahren (a), (b) oder (c) erhaltenen Hydroxyalkinyl-azolyl-Derivate der allgemeinen Formel Ia

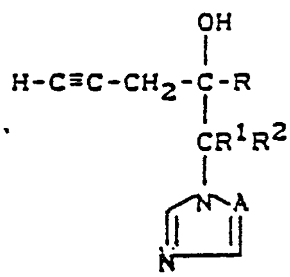

(Ia)

in welcher A, R, $R^1$ und $R^2$ die oben angegebene Bedeutung haben, in an sich bekannter Art und Weise mit Alkalihypohalogenit umsetzt, und gegebenenfalls noch an die so erhaltenen Verbindungen der allgemeinen Formel I eine Säure oder ein Metallsalz addiert.

Schließlich wurde gefunden, daß die neuen Hydroxyalkinylazolyl-Derivate der allgemeinen Formel I sowie deren Säureadditions-Salze und Metallsalz-Komplexe starke fungizide Eigenschaften aufweisen. Dabei zeigen die erfindungsgemäßen Verbindungen überraschenderweise eine bessere fungizide Wirksamkeit als die aus dem Stand der Technik bekannten Hydroxyalkyl-triazole, wie beispielsweise 2-(4-Biphenylyl)-1-(2,4-dichlorphenyl)-3-(1,2,4-triazol-1-yl)-2-propanol oder 2-(4-Biphenylyl)-1-(4-chlorphenyl)-3-(1,2,4-triazol-1-yl)-2-propanol, welche chemisch ähnliche Verbindungen sind. Die erfindungsgemäßen Wirkstoffe stellen somit eine Bereicherung der Technik dar.

Außerdem sind die neuen Hydroxyalkinyl-azolyl-Derivate interessante Zwischenprodukte. So können z. B. die Verbindungen der allgemeinen Formel I an der Hydroxy-Gruppe in üblicher Weise in die entsprechenden Ether übergeführt werden. Weiterhin können durch Umsetzung mit z. B. Acylhalogeniden, Isocyanaten oder Carbamoylchloriden in prinzipiell bekannter Weise Acyl- oder Carbamoyl-Derivate der Verbindungen der allgemeinen Formel I erhalten werden.

Erfindungsgemäße Verbindungen sind auch Additionsprodukte aus Säuren und denjenigen Hydroxyalkinyl-azolyl-Derivaten der allgemeinen Formel I, in denen die Substituenten A, R, $R^1$, $R^2$ und X die Bedeutungen haben, die bereits für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure und Milchsäure, sowie Sulfonsäuren, wie z. B. p-Toluolsulfonsäure und 1,5-Naphthalindisulfonsäure.

Erfindungsgemäße Verbindungen sind außerdem Additionsprodukte aus Salzen von Metalen der II. bis IV. Haupt- und der I. und II. sowie IV. bis VIII. Nebengruppe und denjenigen Hydroxyalkinyl-azolyl-Derivaten der allgemeinen Formel I, in denen die Substituenten A, R, $R^1$, $R^2$ und X die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Verwendet man beispielsweise 5-Chlor-4-(2,4-dichlorphenyl)-1-pentin-4-ol und Imidazol als Ausgangsstoffe, so kann der Ablauf des erfindungsgemäßen Verfahrens (b) durch das folgende Formelschema wiedergegeben werden :

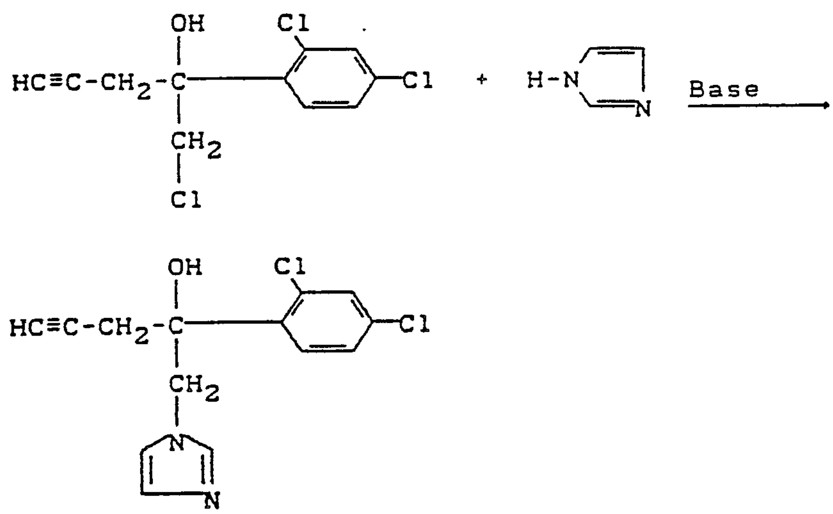


Verwendet man beispielsweise 2-(3,4-Dichlorphenyl)-2-propargyl-oxiran und 1,2,4-Triazol als Ausgangsstoffe, so kann der Ablauf des erfindungsgemäßen Verfahrens (c) durch das folgende Formelschema wiedergegeben werden :

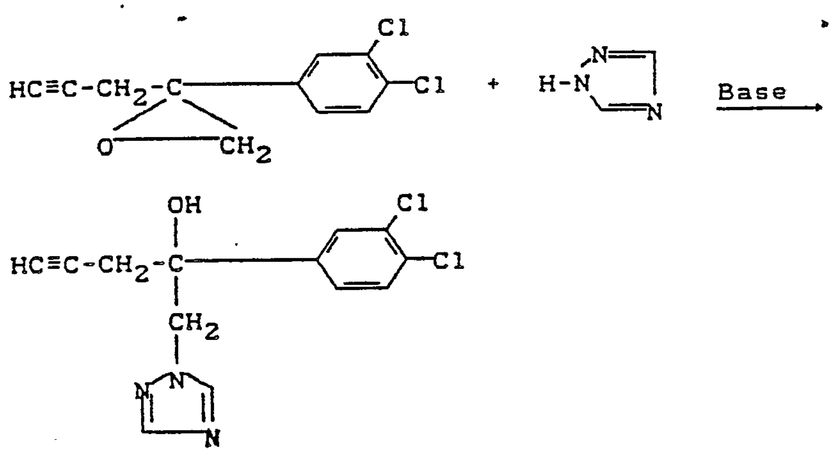


Die bei der Durchführung des erfindungsgemäßen Verfahrens (a) als Ausgangsstoffe zu verwendenden Azolyl-ketone sind durch die allgemeine Formel II allgemeinen definiert. In dieser Formel haben A, R, $R^1$ und $R^2$ diejenigen Bedeutungen, die bereits im Zusammenhang mit der Beschreibung der erfindungsgemäßen Stoffe der allgemeinen Formel I für diese Substituenten genannt wurden.

Die Azolyl-ketone der allgemeinen Formel II sind weitgehend bekannt (vergleiche z. B. DE-A 24 31 407, DE-A 26 10 022, DE-A 26 38 470, DE-A 29 51 164, DE-A 30 48 266, DE-A 31 45 857 und DE-A 31 45 858 ; bzw. können sie in allgemein üblicher Art und Weise erhalten werden.

Die außerdem für das erfindungsgemäße Verfahren (a) als Ausgangsstoffe zu verwendenden Propargylhalogenide der allgemeinen Formel III sind allgemein bekannte Verbindungen der organischen Chemie.

Die bei der Durchführung des erfindungsgemäßen Verfahrens (b) als Ausgangsstoffe zu verwendenden Hydroxyalkinylhalogenide sind durch die allgemeine Formel IV allgemein definiert. In dieser Formel haben R, $R^1$ und $R^2$ die Bedeutungen, die bereits im Zusammenhang mit der Beschreibung der erfindungsgemäßen Stoffe der allgemeinen Formel I für diese Substituenten genannt wurden.

Die Hydroxyalkinyl-halogenide der allgemeinen Formel IV sind noch nicht bekannt ; sie können

jedoch in einfacher Weise erhalten werden, indem man Halogenketone der allgemeinen Formel VII

$$R—CO—CR^1R^2—Hal' \quad (VII)$$

in welcher Hal', R, $R^1$ und $R^2$ die oben angegebene Bedeutung haben, gemäß Verfahren (a) mit Propargylhalogeniden der allgemeinen Formel III umsetzt.

Die Halogenketone der allgemeinen Formel VII sind bekannt; bzw. können sie in allgemein bekannter Art und Weise erhalten werden.

Die bei der Durchführung des erfindungsgemäßen Verfahrens (c) als Ausgangsstoffe zu verwendenden Oxirane sind durch die allgemeine Formel VI allgemein definiert. In dieser Formel haben R, $R^1$ und $R^2$ die Bedeutungen, die bereits im Zusammenhang mit der Beschreibung der erfindungsgemäßen Stoffe der allgemeinen Formel I für diese Substituenten genannt wurden.

Die Oxirane der allgemeinen Formel VI sind noch nicht bekannt; sie können jedoch in einfacher Weise erhalten werden, indem man Hydroxyalkinyl-halogenide der allgemeinen Formel IV mit einer Base, wie beispielsweise Kaliumcarbonat, in einem Zweiphasensystem, wie beispielsweise Methylenchlorid/Wasser, in Gegenwart eines Phasentransferkatalysators, wie beispielsweise Triethylbenzylammoniumchlorid, bei Temperaturen zwischen 20 und 50 °C umsetzt.

Die außerdem für die erfindungsgemäßen Verfahren (b) und (c) als Ausgangsstoffe zu verwendenden Azole sind durch die allgemeine Formel V allgemein definiert. In dieser Formel steht A für die Bedeutungen, die bereits in der Erfindungsdefinition genannt wurden.

Die Azole der allgemeinen Formel V sind allgemein bekannte Verbindungen der organischen Chemie.

Die für das erfindungsgemäße Verfahren (d) als Ausgangsstoffe zu verwendenden Hydroxyalkinyl-azolyl-Derivate der allgemeinen Formel Ia sind erfindungsgemäße Verbindungen.

Als Verdünnungsmittel kommen für das erfindungsgemäße Verfahren (a) organische, aprotische Lösungsmittel in Frage, wie beispielsweise Diethylether oder Tetrahydrofuran.

Die Umsetzung gemäß Verfahren (a) wird in Gegenwart von aktiviertem Aluminium durchgeführt. Dieses wird durch Zugabe katalytischer Mengen Quecksilber (II)-chlorid und Iod erreicht.

Die Reaktionstemperaturen können bei der Durchführung des erfindungsgemäßen Verfahrens (a) in einem größeren Bereich variiert werden. Im allgemeinen arbeitet man bei Temperaturen zwischen —80 und 100 °C, vorzugsweise zwischen —70 und 60 °C.

Bei der Durchführung des erfindungsgemäßen Verfahrens (a) setzt man vorzugsweise auf 1 Mol Azolyl-keton der allgemeinen Formel II 1 bis 2 Mol Propargylhalogenid der allgemeinen Formel III sowie 1 bis 1,5 Mol Aluminium und katalytische Mengen Quecksilber (II)-chlorid und Iod ein. Die Isolierung der Endprodukte erfolgt in allgemein üblicher Weise.

Als Verdünnungsmittel kommen für das erfindungsgemäße Verfahren (b) vorzugsweise polare organische Lösungsmittel in Frage. Hierzu gehören vorzugsweise Alkohole, wie Methanol, Ethanol, Propanol, Isopropanol, Butanol oder Isobutanol; Nitrile, wie Acetonitril oder Propionitril; Amide, wie Dimethylformamid, Formamid oder Dimethylacetamid; und Sulfoxide, wie Dimethylsulfoxid oder Sulfolan.

Als Basen kommen für das erfindungsgemäße Verfahren (b) alle üblicherweise verwendbaren anorganischen und organischen Basen in Betracht. Hierzu gehören vorzugsweise Alkalicarbonate, wie Kaliumcarbonat oder Natriumcarbonat; Alkalialkoholate, wie Natriummethylat und -ethylat oder Kaliummethylat und -ethylat; Alkalihydride, wie Natriumhydrid; tertiäre Amine, wie Triethylamin oder Benzyldimethylamin; sowie auch Azole der Formel allgemeinen V oder deren Alkalisalze.

Die Rekationstemperaturen können bei der Durchführung des erfindungsgemäßen Verfahrens (b) in einem größeren Bereich variiert werden. Im allgemeinen arbeitet man bei Temperaturen zwischen 20 und 150 °C, vorzugsweise zwischen 40 und 120 °C.

Bei der Durchführung des erfindungsgemäßen Verfahrens (b) setzt man vorzugsweise auf 1 Mol Hydroxyalkinyl-halogenid der allgemeinen Formel IV 1 bis 2 Mol Azol der allgemeinen Formel V und 1 bis 4 Mol Base ein. Die Isolierung der Endprodukte erfolgt in allgemein üblicher Weise.

Als Verdünnungsmittel kommen für das erfindungsgemäße Verfahren (c) unter den Reaktionsbedingungen inerte organische Lösungsmittel in Frage. Hierzu gehören vorzugsweise Alkohole, wie Methanol, Ethanol, Propanol, Isopropanol, Butanol oder Isobutanol; Nitrile, wie Acetonitril; Ester, wie Essigester; Ether, wie Dioxan; Amide, wie Dimethylformamid; sowie aromatische Kohlenwasserstoffe, wie Benzol und Toluol.

Als Basen kommen für das erfindungsgemäße Verfahren (c) alle üblicherweise verwendbaren anorganischen und organischen Basen in Betracht. Hierzu gehören vorzugsweise die beim Verfahren (b) bereits vorzugsweise genannten Basen.

Die Reaktionstemperaturen können bei der Durchführung des erfindungsgemäßen Verfahrens (c) in einem größeren Bereich variiert werden. Im allgemeinen arbeitet man bei Temperaturen zwischen 20 und 150 °C, vorzugsweise zwischen 40 und 120 °C.

Bei der Durchführung des erfindungsgemäßen Verfahrens (c) setzt man vorzugsweise auf 1 Mol Oxiran der allgemeinen Formel VI 1 bis 2 Mol Azol der allgemeinen Formel V und gegebenenfalls katalytische bis molare Mengen Base ein. Die Isolierung der Endprodukte erfolgt in algemein üblicher Weise.

Als Verdünnungsmittel kommen für das erfindungsgemäße Verfahren (d) Wasser sowie gegenüber Alkalihypohalogenit inerte organische Lösungsmittel in Frage. Hierzu gehören beispielsweise Alkohole, wie Methanol oder Ethanol; Ether, wie Diethylether, Dioxan oder Tetrahydrofuran; sowie auch Zweiphasengemische, wie z. B. Ether/Wasser.

Die Reaktionstemperaturen können bei der Durchführung des erfindungsgemäßen Verfahrens (d) in einem größeren Bereich variiert werden. Im allgemeinen arbeitet man bei Temperaturen zwischen 0 und 60 °C, vorzugsweise zwischen 0 und 40 °C.

Bei der Durchführung des erfindungsgemäßen Verfahrens (d) setzt man auf 1 Mol der Verbindung der allgemeinen Formel Ia vorzugsweise 1 bis 1,5 Mol Hypohalogenit ein, wobei das Alkalihypohalogenit in situ aus dem entsprechenden Halogen und dem Alkalihydroxid, insbesondere Natrium- und Kaliumhydroxid, erzeugt wird. Die Isolierung der Endprodukte erfolgt in allgemein üblicher Weise.

Zur Herstellung von Säureadditions-Salzen der Verbindungen der allgemeinen Formel I kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der allgemeinen Formel I können in einfacher Weise nach üblichen Salzbildungsmethoden, durch Lösen einer Verbindung der algemeinen Formel I in einem geeigneten Lösungsmittel und Hinzufügen der Säure, z. B. Chlorwasserstoffsäure, erhalten werden und in bekannter Weise, z. B. durch Abfiltrieren isoliert und gegebenenfalls durch Waschen mit einem inerten organischen Lösungsmittel gereinigt werden.

Zur Herstellung von Metallsalz-Komplexen von Verbindungen der allgemeinen Formel I kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Lomplexe von Verbindungen der allgemeinen Formel I können in einfacher Weise nach üblichen Verfahren erhalten werden, so z. B. durch Lösen des Metallsalzes in Alkohol, z. B. Ethanol und Hinzufügen zur Verbindung der allgemeinen Formel I. Man kann Metallsalz-Komplexe in bekannter Weise z. B. durch Abfiltrieren, Isolieren und gegebenenfalls durch Umkristallisieren reinigen.

Die erfindungsgemäßen Wirkstoffe weisen eine starke mikrobiozide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanznschutzmittel können die erfindungsgemäßen Wirkstoffe mit besonders gutem Erfolg zur Bekämpfung von Venturia-Arten, wie gegen den Erreger des Apfelschorfes (Venturia inaequalis), von Erysiphe-Arten, wie gegen den Erreger des echten Gerstenmehltaus (Erysiphe graminis), von weiteren Getreidekrankheiten, wie Cochliobolus sativus und Pyrenophora teres, sowie von Reiskrankheiten, wie Pyricularia oryzae und Pellicularia sasakii, eingesetzt werden. Hervorzuheben ist die zusätzliche bakterizide Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-konzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor,

Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycolether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel, wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe, wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmiteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlammbeizen, oder Inkrustieren.

Bei der Behandlung von Pflanzenteilen können die Wirkstoffkonzentrationen in den Anwendungsformen in einem größeren Bereich variiert werden. Sie liegen in allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

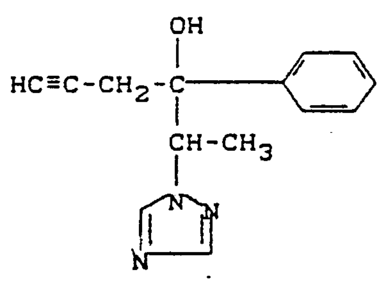

(Verfahren a)

6,5 g (0,241 Mol) Aluminium (in Schuppenform), eine Spatelspitze Quecksilber (II)-chlorid und ein Iodkristall werden zusammen mit 35 ml Tetrahydrofuran 12 Stunden gerührt. Dann werden bei 50 bis 60 °C 42 g (0,35 Mol) Propargylbromid in 50 ml Tetrahydrofuran zugetropft. Man rührt noch 30 Minuten bei 60 °C, kühlt auf — 60 °C ab und tropft bei dieser Temperatur langsam 34,2 g (0,17 Mol) 2-(1,2,4-Triazol-1-yl)-propiophenon in 150 ml Tetrahydrofuran zu. Nach Beendigung der Zugabe rührt man noch 1 Stunde bei 0 °C und 2 Stunden bei Raumtemperatur, tropft unter Außenkühlung 85 ml gesättigte, wäßrige Ammoniumchlorid-Lösung zu, filtriert und engt das Filtrat im Vakuum ein. Der Rückstand wird dreimal mit je 150 ml Essigester/Toluol (1 : 1) extrahiert, die vereinigten organischen Phasen werden mit Wasser gewaschen, über wasserfreiem Natriumsulfat getrocknet und im Vakuum eingeengt. Nach dem Verrühren des festen Rückstandes mit 40 ml Toluol erhält man 18,7 g (45,6 % der Theorie) 4-Phenyl-5-(1,2,4-triazol-1-yl)-1-hexin-4-ol in Form farbloser Kristalle vom Schmelzpunkt 123 °C.

Herstellung des Vorproduktes

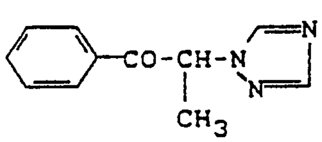

Man tropft zu einer Lösung von 223 g (1 Mol) 2-Brompropiophenon in 300 ml Acetonitril zu einer siedenden Mischung aus 414 g (6 Mol) Triazol und 1 200 ml Acetonitril und erhitzt 8 Stunden zum Sieden. Anschließend engt man im Vakuum ein, nimmt der Rückstand in 750 ml Dichlormethan und 750 ml Wasser auf, wäscht die organische Phase mehrmals mit je 50 ml Wasser und trocknet über wasserfreiem Natriumsulfat. Nach dem Abdestillieren des Lösungsmittels wird der verbleibende Rückstand im Vakuum destilliert. Man erhält 111 g (55 % der Theorie) 2-(1,2,4-Triazol-1-yl)-propiophenon vom Siedepunkt 133 °C/0,15 mbar und vom Schmelzpunkt 117 °C.

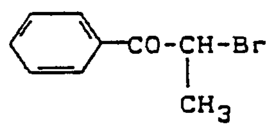

Zu einer Lösung von 72 g (0,5 Mol) Propiophenon in 500 ml Dichlormethan tropft man bei 20 °C eine Lösung von 82 g (0,51 Mol) Brom in 100 ml Dichlormethan zu. Nach 30 Minuten wird die Lösung eingeengt. Man erhält quantitativ 2-Brompropiophenon, das direkt weiter umgesetzt wird.

Beispiel 2

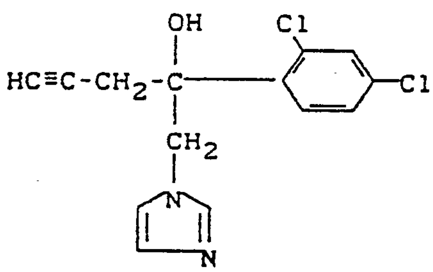

(Verfahren b)

Zu einer siedenden Lösung von 40,8 g (0,6 Mol) Imidazol in 250 ml n-Propanol werden 26,4 g (0,1 Mol) 5-Chlor-4-(2,4-dichlorphenyl)-1-pentin-4-ol, gelöst in 30 ml n-Propanol, zugetropft. Nach 48 Stunden wird die Lösung im Vakuum eingeengt, der ölige Rückstand in 150 ml Essigester aufgenommen und die organische Phase dreimal mit je 50 ml Wasser gewaschen. Durch Einengen der Lösung erhält man 19 g (64,4 % der Theorie) an 4-(2,4-Dichlorphenyl)-5-imidazol-1-yl-1-pentin-4-ol als farblose Kristalle vom Schmelzpunkt 179-180 °C.

Herstellung des Vorproduktes

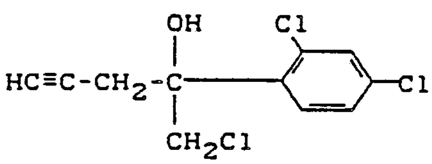

13 g (0,48 Mol) Aluminium (in Schuppenform) werden mit einer Spatelspitze Quercksilber (II)-chlorid und einem Iodkristall versetzt, mit 60 ml Tetrahydrofuran überschichtet und 10 Stunden bei 20 °C gerührt. Man erwärmt anschließend auf 60 °C tropft eine Lösung von 84,5 g (0,71 Mol) Propargylbromid in 85 ml Tetrahydrofuran zu. Das Gemisch wird dan auf — 60 °C gekühlt und eine Lösung von 111,7 g (0,5 Mol) ω-Chlor-2,4-dichloracetophenon in 165 ml Tetrahydrofuran eingetropft. Man läßt auf 0 °C erwärmen und hydrolysiert nach 1 Stunde mit 170 ml gesättigter, wäßriger Ammoniumchlorid-Lösung.

Anschließend wird filtriert, das Filtrat im Vakuum eingeengt und mit 500 ml Essigester versetzt. Man trennt die organische Phase ab, wäscht dreimal mit je 300 ml Wasser, trocknet über wasserfreiem Natriumsulfat und destilliert das Lösungsmittel unter vermindertem Druck ab. Man erhält 113,5 g (86,1 % der Theorie) 5-Chlor-4-(2,4-dichlorphenyl)-1-pentin-4-ol als farblose Kristalle vom Schmelzpunkt 55-57 °C.

Beispiel 3

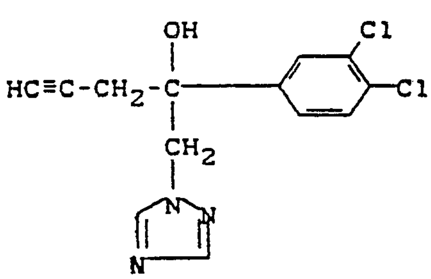

(Verfahren c)

Zu einer siedenden Lösung von 8,05 g (0,116 Mol) 1,2,4-Triazol in 400 ml n-Butanol werden 0,275 g (0,011 Mol) Natrium eingetragen. Anschließend werden 24 g (0,105 Mol) 2-(3,4-Dichlorphenyl)-2-propargyl-oxiran, gelöst in 50 ml n-Butanol, zugegeben. Man erhitzt die Lösung 12 Stunden zum Sieden, destilliert das Lösungsmittel im Vakuum ab, nimmt in 200 ml Trichlormethan auf, wäscht zweimal mit je 50 ml Wasser, trocknet die organische Phase über wasserfreiem Natriumsulfat und filtriert anschließend über 200 g Kiesselgel. Man erhält 18,45 g (53,6 % der Theorie) 4-(3,4-Dichlorphenyl)-5-(1,2,4-triazol-1-yl)-1-pentin-4-ol als farblose Kristalle vom Schmelzpunkt 114 °C.

Herstellung des Vorproduktes

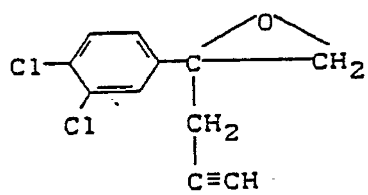

60,3 g (0,196 Mol) 5-Brom-4-(3,4-dichlorphenyl)-1-propin-4-ol werden zusammen mit 17 g Triethylbenzylammonium-chlorid und 460 g Kaliumcarbonat in einem Zweiphasensystem aus 1 800 ml Dichlormethan und 850 ml Wasser für 8 Stunden bei 20 bis 25 °C gerührt. Man gibt anschließend 500 ml Tetrachlormethan zu, trennt die organische Phase ab und wäscht mehrmals mit Wasser. nach dem Einengen im Vakuum wird der ölige Rückstand über 200 g Kieselgel (Laufmittel: Tetrachlormethan) filtriert und das Filtrat unter vermindertem Druck eingedampft. Es werden 38 g (86 % der Theorie) 2-(3,4-Dichlorphenyl)-2-propargyl-oxiran vom Brechungsindex $n^{20}_D$ = 1,5676 erhalten.

In analoger Weise und entsprechend der erfindungsgemäßen Verfahren (a) bis (d) werden die nachfolgenden Verbindungen der allgemeinen Formel I

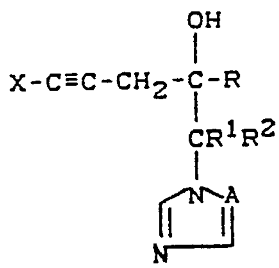

(I)

erhalten:

| Bsp. | R | $R^1$ | $R^2$ | X | A | Schmelzp. (°C bzw. Brechungsindex ($n_D^{20}$)) |
|---|---|---|---|---|---|---|
| 4 | 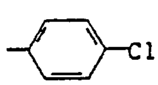 | H | H | H | N | 142 |
| 5 | 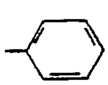 | H | H | H | N | 1,5745 |
| 6 | 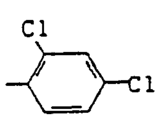 | H | H | H | N | 169-70 |
| 7 | 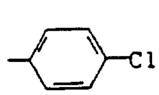 | H | H | H | CH | 120-21 |
| 8 | 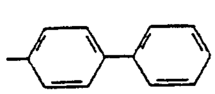 | H | H | H | CH | 117-18 |

(Fortsetzung)

| Bsp. | R | $R^1$ | $R^2$ | X | A | Schmelzp. (°C bzw. Brechungsindex ($n_D^{20}$)) |
|---|---|---|---|---|---|---|
| 9 | 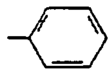 | H | H | H | CH | 113 |
| 10 | 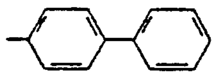 | H | H | H | N | Harz |
| 11 | 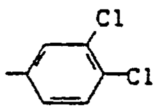 | H | H | H | CH | 116 |
| 12 | 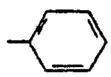 | $CH_3$ | H | H | CH | zähes Öl |
| 13 | 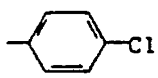 | H | H | I | N | 108-09 |
| 14 | 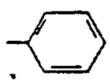 | H | H | I | N | Harz |
| 15 | 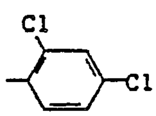 | H | H | I | CH | 171-72 |
| 16 | 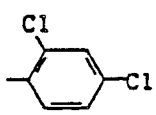 | H | H | I | N | 156 |
| 17 | 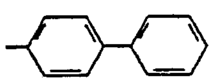 | H | H | I | CH | 114 |
| 18 | 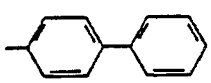 | H | H | I | N | 104-05 |
| 19 | 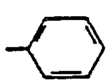 | H | H | I | CH | 93 |
| 20 | 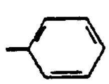 | $-CH_3$ | H | I | CH | 159 |
| 21 | 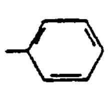 | $-CH_3$ | H | I | N | 112 |

(Fortsetzung)

| Bsp. | R | $R^1$ | $R^2$ | X | A | Schmelzp. (°C bzw. Brechungsindex ($n_D^{20}$)) |
|---|---|---|---|---|---|---|
| 22 | 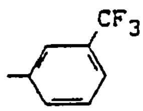 | H | H | H | CH | 124-27 |
| 23 | 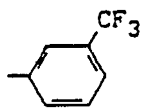 | H | H | H | N | Öl |
| 24 | 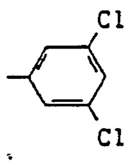 | H | H | H | CH | 105-07 |
| 25 | 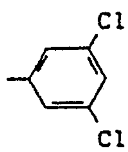 | H | H | H | N | 138 |

## Verwendungsbeispiele

In den nachfolgenden Verwendungsbeispielen werden die nachstehend angegebenen Substanzen als Vergleichsverbindungen eingesetzt:

(A)

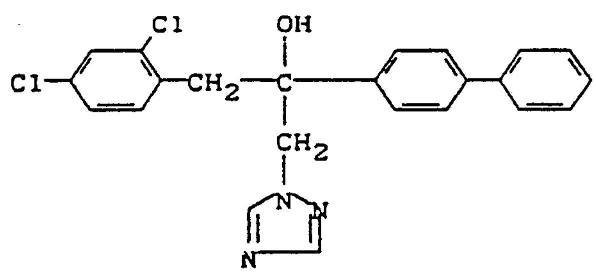

(B)

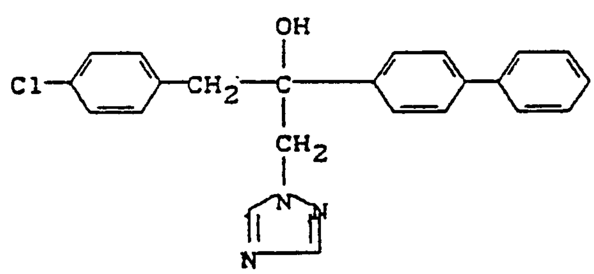

## Beispiel A

Venturia-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann in Gewäckshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z. B. die Verbindung gemäß Herstellungsbeispiel 4.

Beispiel B

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehlaupusteln zu begünstigen.

7 Tage nach der Inokulation erfolgt die Auswertung.

Eine deutliche Überlegenheit in der Wirksamkeit gegenüber dem Stand der Technik zeigen in diesem Test z. B. die Verbindungen gemäß Herstellungsbeispiele : 4, 6, 7, 9.

Beispiel C

Pellicularia-Test (Reis)

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen in Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z. B. die Verbindungen gemäß Herstellungsbeispiele : 14, 4, 6, 15.

**Patentansprüche**

1. Hydroxyalkinyl-azolyl-Derivate der allgemeinen Formel I

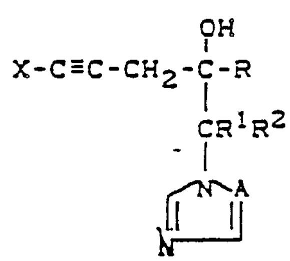

(I)

in welcher

A für ein Stickstoffatom oder eine CH-Gruppe steht,

R für Phenyl steht, das gegebenenfalls einfach bis zweifach, gleich oder verschieden substituiert ist durch Chlor, Methyl, Trifluormethyl sowie gegebenenfalls durch Chlor substituiertes Phenyl,

$R^1$ für Wasserstoff, Methyl, Vinyl, Allyl, Propargyl sowie für Benzyl steht, das im Phenylteil gegebenenfalls einfach bis zweifach, gleich oder verschieden substituiert ist durch Fluor, Chlor, Brom, Methyl, Ethyl, Isopropyl, tert.-Butyl, Methoxy, Methylthio, Ethoxy, Trifluormethyl, Trifluormethoxy, Trifluormethylthio, Nitro, Dimethylamino, Methoxycarbonyl sowie gegebenefalls durch Chlor substituiertes Phenyl,

$R^2$ für Wasserstoff oder Methyl steht und

X für Wasserstoff, Brom oder Jod steht,

sowie deren Säureadditions-Salze und Metallsalz-Komplexe.

2. Verfahren zur Herstellung von Hydroxyalkinyl-azolyl-Derivaten der allgemeinen Formel I

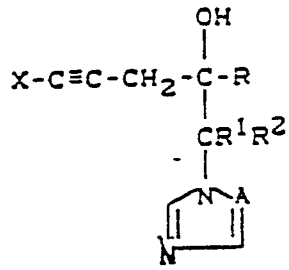

(I)

in welcher

A für ein Stickstoffatom oder eine CH-Gruppe steht,

R für Phenyl steht, das gegebenenfalls einfach bis zweifach, gleich oder verschieden substituiert ist durch Chlor, Methyl, Trifluormethyl sowie gegebenenfalls durch Chlor substituiertes Phenyl,

$R^1$ für Wasserstoff, Methyl, Vinyl, Allyl, Propargyl sowie für Benzyl steht, das im Phenylteil gegebenenfalls einfach bis zweifach, gleich oder verschieden substituiert ist durch Fluor, Chlor, Brom, Methyl, Ethyl, Isopropyl, tert.-Butyl, Methoxy, Methylthio, Ethoxy, Trifluormethyl, Trifluormethoxy, Trifluormethylthio, Nitro, Dimethylamino, Methoxycarbonyl sowie gegebenenfalls durch Chlor substituiertes Phenyl,

$R^2$ für Wasserstoff oder Methyl steht und

X für Wasserstoff, Brom oder Jod steht,

sowie von deren Säureadditions-Salzen und Metallsalz-Komplexen, dadurch gekennzeichnet, daß man

a) Azolyl-ketone der allgemeinen Formel II

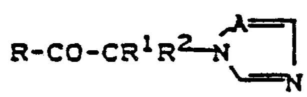

(II)

in welcher A, R, $R^1$ und $R^2$ die oben angegebene Bedeutung haben, mit Propargylhalogeniden der allgemeinen Formel III

$$H - C \equiv C - CH_2 - Hal \quad (III)$$

in welcher Hal für Chlor oder Brom steht, in Gegenwart von aktiviertem Aluminium und in Gegenwart eines Verdünnungsmittels umsetzt, oder

b) Hydroxyalkinyl-halogenide der allgemeinen Formel IV

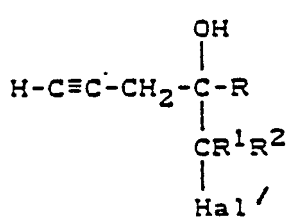

(IV)

in welcher

R, $R^1$ und $R^2$ die oben angegebene Bedeutung haben und

Hal' für Chlor oder Brom steht,

mit Azolen der allgemeinen Formel V

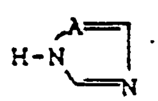

(V)

in welcher A die oben angegebene Bedeutung hat, in Gegenwart einer Base und in Gegenwart eines Verdünnungsmittels umsetzt, oder

c) Oxirane der allgemeinen Formel VI

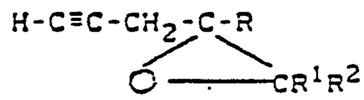

(VI)

in welcher R, $R^1$ und $R^2$ die oben angegebene bedeutung haben, mit Azolen der allgemeinen Formel V

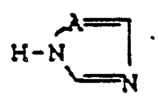

(V)

in welcher A die oben angegebene Bedeutung hat, in Gegenwart eines Verdünnungsmittels und gegebenenfalls in Gegenwart einer Base umsetzt, oder

d) gegebenenfalls die nach den Verfahren (a), (b) oder (c) erhaltenen Hydroxyalkinyl-azolyl-Derivate der allgemeinen Formel Ia

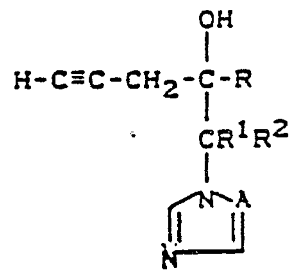

(Ia)

in welcher A, R, $R^1$ und $R^2$ die oben angegebene Bedeutung haben, in an sich bekannter Art und Weise mit Alkalihypohalogenit umsetzt, und gegebenenfalls noch an die so erhaltenen Verbindungen der allgemeinen Formel I eine Säure oder ein Metallsalz addiert.

3. Fungizide Mittel, gekennzeichnet durch einen Gehalt an mindestens einem Hydroxyalkinyl-azolyl-Derivat der allgemeinen Formel I gemäß Anspruch 1 bzw. einem säureadditions-Salz oder Metallsalz-Komplex eines Hydroxyalkinyl-azolyl-Derivates der allgemeinen Formel I.

4. Verfahren zur Bekämpfung von Pilzen, dadurch gekennzeichnet, daß man Hydroxyalkinyl-azolyl-Derivate der allgemeinen Formel I gemäß Anspruch 1 bzw. deren Säureadditions-Salze oder Metallsalz-Komplexe auf Pilze oder ihren Lebensraum einwirken läßt.

5. Verwendung von Hydroxyalkinyl-azolyl-Derivaten der allgemeinen Formel I gemäß Anspruch 1 bzw. von deren Säureadditions-Salzen oder Metallsalz-Komplexen zur Bekämpfung von Pilzen.

6. Verfahren zur Herstellung von fungiziden Mitteln, dadurch gekennzeichnet, daß man Hydroxyalkinylazolyl-Derivate der allgemeinen Formel I gemäß Anspruch 1 bzw. deren Säureadditons-Salze oder Metallsalz-Komplexe mit Streckmitteln und/oder oberflächenaktiven Mitteln vermischt.

**Claims**

1. Hydroxyalkinyl-azolyl derivatives of the general formula I

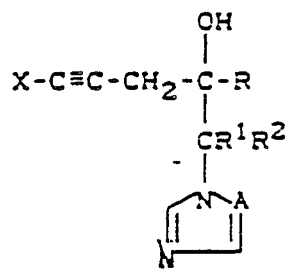

(I)

in which

A represents a nitrogen atom or a CH group,

R represents phenyl which is optionally monosubstituted to disubstituted by identical or different substituents from amongst chlorine, methyl, trifluoro-methyl and also optionally chlorine-substituted phenyl,

$R^1$ represents hydrogen, methyl, vinyl, allyl propargyl or benzyl which is optionally monosubstituted to disubstituted in the phenyl part by identical or different substituents from amongst fluorine, chlorine, homine, methyl, ethyl, isopropyl, test-butyl, methoxy, methylthio, ethoxy, trifluoromethyl, trifluoromethoxy, trifluoromethylthion nitro, dimethylamino, methoxycarbonyl and also by optionally chlorine-substituted phenyl,

$R^2$ represents hydrogen or methyl and

X represents hydrogen, bromine or iodine,

and their acid addition salts and methal salt complexes.

2. Process for the preparation of hydroxyalkinylazolyl derivatives of the general formula I

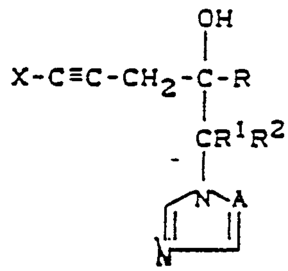

(I)

in which

A represents a nitrogen atom or a CH group,

R represents phenyl which is optionally monosubstituted to disubstituted by identical or different substituents from amongst dilorine, methyl trifluoro-methyl. and also optionally chlorine-substituted phenyl,

$R^1$ represents hydrogen, methyl, vinyl, allyl, propargyl or benzyl which is optionally monosubstituted to disubstituted in the phenyl part by identical or different substituents from amongst fluorine, chlorine, bromine, methyl, ethyl, isopropyl, test-butyl, methoxy, methylthio, ethoxy, trifluoromethyl, trifluoromethoxy, trifluoromethylthio, nitro, dimethylamino, methoxycarbonyl and optionally chlorine-substituted phenyl,

$R^2$ represents hydrogen or methyl and

X represents hydrogen, bromine or iodine, and of their addition salts and metal salt complexes, characterized in that

a) azolyl-ketones of the general formula II

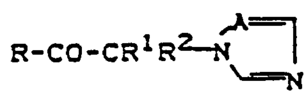

(II)

in which A, R, $R^1$ and $R^2$ have the meaning given above, are reacted with propargyl halides of the general formula III

$$H—C \equiv C—CH_2—Hal \qquad (III)$$

in which Hal represents chlorine or bromine, in the presence of activated aluminium and in the presence of a diluent, or

b) hydroxyalkinyl halides of the general formula IV

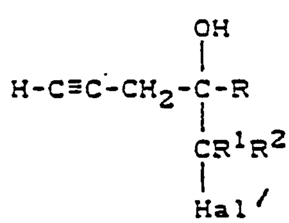

(IV)

in which

R, $R^1$ and $R^2$ have the meaning given above, and

Hal' represents chlorine or bromine,

are reacted with azoles of the general formula V

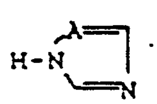

(V)

in which A has the meaning given above, in the presence of a base and in the presence of a diluent, or

c) oxiranes of the general formula VI

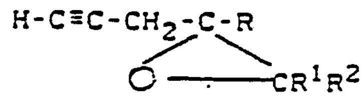

(VI)

in which R, $R^1$ and $R^2$ have the meaning given above, are reacted with azoles of the general formula V

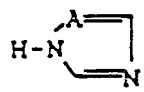

(V)

in which A has the meaning given above, in the presence of a diluent and, if appropriate, in the presence of a base, or

d) if appropriate, the hydroxyalkinyl-azolyl derivatives obtained by processes (a), (b) or (c), of the general formula Ia

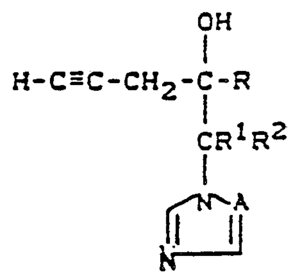

(Ia)

in which A, R, $R^1$ and $R^2$ have the meaning given above, are reacted with an alkali metal hypohalite in a manner which is in itself known and, if desired, the resulting compounds of the general formula I are further subjected to an addition reaction with an acid or a metal salt.

3. Fungicidal agents, characterized in that they contain at least one hydroxyalkinyl-azolyl derivative of the general formula (I) according to Claim 1 or an acid addition salt or a metal salt complex of a hydroxy alkinyl-azolyl derivative of the general formula I.

4. Process for combating fungi, characterized in that hydroxyalkinyl-azolyl derivatives of the general formula I according to Claim 1 or their acid addition salts or metal salt complexes are allowed to act on fungi or their habitat.

5. Use of hydroxyalkinyl-azolyl derivatives of the general formula I according to Claim 1 or of their acid additions salts or metal salt complexes for combating fungi.

6. Process for the preparation of fungicidal agents, characterized in that hydroxyalkinyl-azolyl derivatives of the general formula I according to Claim 1 or their acid addition salts or metal salt complexes are mixed with extenders and/or surface active agents.

**Revendications**

1. Dérivés d'hydroxyalcynyl-azolyle de formule générale I :

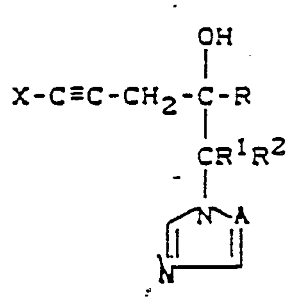

(I)

dans laquelle

A représente un atome d'azote ou un groupe CH,

R est un groupe phényl qui porte le cas échéant un ou deux substituants, identiques ou différents, chloro, méthyle, trifluorométhyle ainsi que, le cas échéant, phényle substitué par du chlore,

$R^1$ est l'hydrogène, un groupe méthyle, vinyle, allyle, propargyle de même qu'un groupe benzyle qui porte éventuellement dans la partie phényle un ou deux substituants, identiques ou différents, fluoro,

chloro, bromo, méthyle, éthyle, isopropyle, tertio-butyle, méthoxy, méthylthio, éthoxy, trifluorométhyle, trifluorométhoxy, trifluorométhylthio, nitro, diméthylamino, méthoxycarbonyle, ainsi que phényle éventuellement substitué par du chlore,

$R^2$ est l'hydrogène ou le groupe méthyle et

X est l'hydrogène, le brome ou l'iode,

ainsi que leurs sels d'addition d'acides et leurs complexes de sels métalliques.

2. Procédé de production de dérivés d'hydroxyalcynyl-azoyle de formule générale I

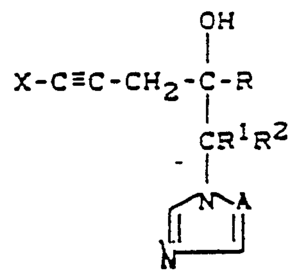

(I)

dans laquelle

A représente un atome d'azote ou un groupe CH,

R est un groupe phényle qui porte le cas échéant un ou deux substituants, identiques ou différents, chloro, méthyle, trifluorométhyle ainsi que, le cas échéant, phényle substitué par du chlore,

$R^1$ est l'hydrogène, un groupe méthyle, vinyle, allyle, propargyle de même qu'un groupe benzyle qui porte éventuellement dans la partie phényle un ou deux substituants, identiques ou différents, fluoro, chloro, bromo, méthyle, éthyle, isopropyle, tertio-butyle, méthoxy, méthylthio, éthoxy, trifluorométhyle, trifluorométhoxy, trifluorométhylthio, nitro, diméthylamino, méthoxycarbonyle, ainsi que phényle éventuellement substitué par du chlore,

$R^2$ est l'hydrogène ou le groupe méthyle et

X est l'hydrogène, le brome ou l'iode,

ainsi que de leurs sels d'addition d'acides et de leurs complexes de sels métalliques, caractérisé en ce que :

a) on fait réagir des azolyl-cétones de formule générale II

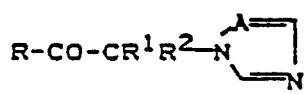

(II)

dans laquelle A, R, $R^1$ et $R^2$ ont la définition indiquée ci-dessus, avec des halogénures de propargyle de formule générale III

$$H—C \equiv CH_2—Hal \quad (III)$$

dans laquelle Hal représente le chlore ou le brome, en présence d'aluminium activé et en présence d'un diluant, ou bien

b) on fait réagir des halogénures d'hydroxy-alcynyle de formule générale IV

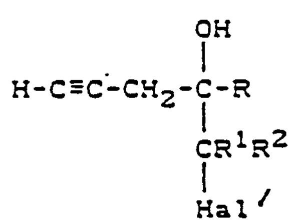

(IV)

dans laquelle

R, $R^1$ et $R^2$ ont la définition indiquée ci-dessus, et

Hal' représente le chlore ou le brome,

avec des azoles de formule générale V

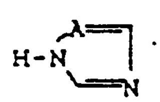

(V)

dans laquelle A a la définition indiquée ci-dessus, en présence d'une base et en présence d'un diluant, ou bien

c) on fait réagir des oxirannes de formule générale VI

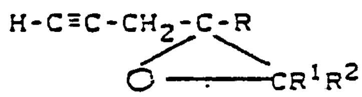

(VI)

dans laquelle R, $R^1$ et $R^2$ ont la définition indiquée ci-dessus, avec des azoles de formule générale V

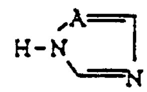

(V)

dans laquelle A a la définition indiquée ci-dessus, en présence d'un diluant et le cas échéant en présence d'une base, ou bien

d) on fait réagir éventuellement les dérivés d'hydroxyalcynyl-azolyle obtenus selon les procédés (a), (b) ou (c), de formule générale Ia

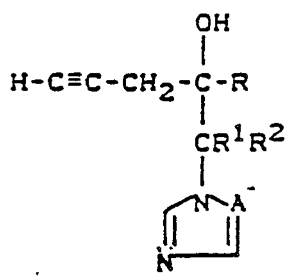

(Ia)

dans laquelle A, R, $R^1$, et $R^2$ ont la définition indiquée ci-dessus, d'une manière connue avec un hypohalogénite alcalin, et on additionne encore éventuellement sur les composés ainsi obtenus de formule générale (I) un acide ou un sel métallique.

3. Compositions fongicides, caractérisées par une teneur en au moins un dérivé d'hydroxyalcynyl-azolyle de formule générale I suivant la revendication 1 ou en un sel d'addition d'acide ou en un complexe de sel métallique d'un dérivé d'hydroxyalcynyl-azolyle de formule générale I.

4. Procédé pour combattre des champignons, caractérisé en ce qu'on fait agir des dérivés d'hydroxyalcynyl-azolyle de formule I suivant la revendication 1 ou leurs sels d'addition d'acides ou leurs complexes de sels métalliques sur des champignons ou sur leur milieu.

5. Utilisation de dérivés d'hydroxyalcynyl-azolyle de formule générale I suivant la revendication 1 ou de leurs sels d'addition d'acides ou de leurs complexes de sels métalliques pour combattre des champignons.

6. Procédé de préparation de compositions fongicides, caractérisé en ce qu'on mélange des dérivés d'hydroxyalcynyl-azolyle de formule générale I suivant la revendication 1 ou leurs sels d'addition d'acides ou leurs complexes de sels métalliques, avec des diluants et/ou des agents tensio-actifs.